(19) Europäisches Patentamt
European Patent Office
Office européen des brevets

(11) EP 4 703 461 A1

(12) EUROPEAN PATENT APPLICATION

(43) Date of publication:
**04.03.2026 Bulletin 2026/10**

(21) Application number: **24197310.6**

(22) Date of filing: **29.08.2024**

(51) International Patent Classification (IPC):
***C11D 1/83*** *(2006.01)* ***C11D 3/386*** *(2006.01)*
***C11D 17/04*** *(2006.01)* *C11D 1/14 (2006.01)*
*C11D 1/22 (2006.01)* *C11D 1/29 (2006.01)*
*C11D 1/72 (2006.01)*

(52) Cooperative Patent Classification (CPC):
**C11D 3/38618; C11D 1/83; C11D 17/043;**
C11D 1/146; C11D 1/22; C11D 1/29; C11D 1/72

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **The Procter & Gamble Company**
**Cincinnati, OH 45202 (US)**

(72) Inventors:
- **ANDRIESSEN, Hilde Francoise Louse**
**1853 Strombeek-Bever (BE)**
- **DEPOOT, Karel Jozef Maria**
**1853 Strombeek-Bever (BE)**
- **DRAPER, Karolina Anna**
**Newcastle-upon-Tyne, NE12 9TS (GB)**
- **YALDIZKAYA, Nuray**
**1853 Strombeek-Bever (BE)**

(74) Representative: **P&G Patent Belgium UK**
**N.V. Procter & Gamble Services Company S.A.**
**Temselaan 100**
**1853 Strombeek-Bever (BE)**

# (54) WATER-SOLUBLE UNIT DOSE ARTICLE COMPRISING A METALLOPROTEASE

(57) A water-soluble unit dose detergent article comprising a water-soluble film and a laundry liquid detergent composition, wherein the detergent composition comprises:
a non-soap surfactant system comprising anionic non-soap surfactant and nonionic surfactant wherein the anionic non-soap surfactant comprises more than 0% and less than 20% by weight of the anionic non-soap surfactant of an anionic non-soap surfactant selected from the group consisting of alkyl sulphate, alkoxylated alkyl sulphate, and a mixture thereof;
a metalloprotease; and
up to 15% by weight of the composition of water.

EP 4 703 461 A1

## Description

FIELD OF THE INVENTION

Water-soluble unit dose article comprising a detergent composition comprising a surfactant system and a metalloprotease.

BACKGROUND OF THE INVENTION

Water-soluble unit dose detergent articles are liked by consumers as they are convenient and efficient to use. Such water-soluble unit dose articles comprise detergent compositions enveloped by a water-soluble film. When the water-soluble unit dose detergent article is added to water, the film dissolves/disintegrates releasing the detergent into the surrounding water to create a cleaning liquor.

The present invention addresses the challenge of achieving effective fabric cleaning performance while reducing the reliance on ethoxylated alkyl sulphate. Ethoxylated alkyl sulphate is a commonly used surfactant that aids in the removal of dirt, stains, and greasy residues. The present invention seeks alternative compositions comprising lower levels of ethoxylated alkyl sulfate.

Thus, the objective of the present invention is to provides a novel fabric cleaning composition with a reduced level of ethoxylated alkyl sulphate, that offers good soil and stain removal.

SUMMARY OF THE INVENTION

A water-soluble unit dose detergent article comprising a water-soluble film and a liquid detergent composition, wherein the detergent composition comprises:

a non-soap surfactant system, the surfactant system comprising anionic non-soap surfactant and nonionic surfactant. The anionic non-soap surfactant comprises more than 0% and less than 20% by weight of the anionic non-soap surfactant of an anionic non-soap surfactant selected from the group consisting of alkyl sulphate, alkoxylated alkyl sulphate, and a mixture thereof;
up to 15% by weight of the composition of water; and
a metalloprotease.

The water-soluble unit dose detergent article of the invention comprises a cleaning composition that maintains high cleaning efficacy while significantly reducing the amount of ethoxylated alkyl sulphate used.

DETAILED DESCRIPTION OF THE INVENTION

<u>Water-soluble unit dose article</u>

The present invention discloses a water-soluble unit dose article comprising a water-soluble film and a liquid detergent composition, preferably a liquid laundry detergent composition. The water-soluble film and the detergent composition are described in more detail below.

The water-soluble unit dose detergent article comprises the water-soluble film shaped such that the unit-dose article comprises at least one internal compartment surrounded by the water-soluble film. The unit dose article may comprise a first water-soluble film and a second water-soluble film sealed to one another such to define the internal compartment. The water-soluble unit dose article is constructed such that the detergent composition does not leak out of the compartment during storage. However, upon addition of the water-soluble unit dose article to water, the water-soluble film dissolves and releases the contents of the internal compartment into the wash liquor.

The compartment should be understood as meaning a closed internal space within the unit dose article, which holds the detergent composition. During manufacture, a first water-soluble film may be shaped to comprise an open compartment into which the detergent composition is added. A second water-soluble film is then laid over the first film in such an orientation as to close the opening of the compartment. The first and second films are then sealed together along a seal region.

The unit dose article may comprise more than one compartment, even at least two compartments, or even at least three compartments, or even at least four compartments. The compartments may be arranged in superposed orientation, i.e. one positioned on top of the other. In such an orientation the unit dose article will comprise at least three films, top, one or more middle, and bottom. Alternatively, the compartments may be positioned in a side-by-side orientation, i.e. one orientated next to the other. The compartments may even be orientated in a ‘tyre and rim’ arrangement, i.e. a first

compartment is positioned next to a second compartment, but the first compartment at least partially surrounds the second compartment but does not completely enclose the second compartment. Alternatively, one compartment may be completely enclosed within another compartment.

Wherein the unit dose article comprises at least two compartments, one of the compartments may be smaller than the other compartment. Wherein the unit dose article comprises at least three compartments, two of the compartments may be smaller than the third compartment, and preferably the smaller compartments are superposed on the larger compartment. The superposed compartments preferably are orientated side-by-side. The unit dose article may comprise at least four compartments, three of the compartments may be smaller than the fourth compartment, and preferably the smaller compartments are superposed on the larger compartment. The superposed compartments preferably are orientated side-by-side.

In a multi-compartment orientation, the detergent composition according to the present invention may be comprised in at least one of the compartments. It may for example be comprised in just one compartment, or may be comprised in two compartments, or even in three compartments, or even in four compartments. Alternatively the detergent composition according to the invention may be split over different compartments, such that the individual components get combined in the wash solution upon use. For example one compartment may comprise the non-soap surfactant system according to the invention, another compartment may comprise the metalloprotease according to the invention.

Each compartment may comprise the same or different compositions. The different compositions could all be in the same form, or they may be in different forms.

The water-soluble unit dose article may comprise at least two internal compartments, wherein the laundry detergent composition is comprised in at least one of the compartments, preferably wherein the unit dose article comprises at least three compartments, wherein the detergent composition is comprised in at least one of the compartments.

The water-soluble unit dose article may comprise from 1 gram up to 60 gram, preferably from 5 gram up to 50 gram, more preferably from 10 gram up to 40 gram, most preferably from 12 gram up to 25 gram alternatively from 30 gram to 40 gram of the laundry detergent composition. The water-soluble unit dose article may comprise from 1 ml up to 60 ml, preferably from 5 ml up to 50 ml, more preferably from 10 ml up to 40 ml, most preferably from 12 ml up to 25 ml, alternatively from 30 ml to 40ml of the liquid laundry detergent composition.

<u>Water-soluble film</u>

The film of the present invention is soluble or dispersible in water. The water-soluble film preferably has a thickness of from 20 to 150 micron, preferably 35 to 125 micron, even more preferably 50 to 110 micron, most preferably about 76 micron.

Preferably, the film has a water-solubility of at least 50%, preferably at least 75% or even at least 95%, as measured by the method set out here after using a glass-filter with a maximum pore size of 20 microns:
5 grams ± 0.1 gram of film material is added in a pre-weighed 3L beaker and 2L ± 5ml of distilled water is added. This is stirred vigorously on a magnetic stirrer, Labline model No. 1250 or equivalent and 5 cm magnetic stirrer, set at 600 rpm, for 30 minutes at 30°C. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). Then, the percentage solubility or dispersability can be calculated.

Preferred film materials are preferably polymeric materials. The film material can, for example, be obtained by casting, blow-moulding, extrusion or blown extrusion of the polymeric material, as known in the art.

Preferred polymers, copolymers or derivatives thereof suitable for use as pouch material are selected from polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatine, natural gums such as xanthum and carragum. More preferred polymers are selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and most preferably selected from polyvinyl alcohols, polyvinyl alcohol copolymers and hydroxypropyl methyl cellulose (HPMC), and combinations thereof. Preferably, the level of polymer in the pouch material, for example a PVA polymer, is at least 60%. The polymer can have any weight average molecular weight, preferably from about 1000 to 1,000,000, more preferably from about 10,000 to 300,000 yet more preferably from about 20,000 to 150,000.

Preferably, the water-soluble film comprises polyvinylalcohol polymer, preferably wherein the polyvinylalcohol polymer comprises polyvinyl alcohol homopolymer or polyvinyl alcohol copolymer, or a mixture thereof, preferably a blend of polyvinylalcohol homopolymers and/or polyvinylalcohol copolymers, preferably wherein the polyvinylalcohol copolymers are selected from sulphonated and carboxylated anionic polyvinylalcohol copolymers especially carboxylated anionic polyvinylalcohol copolymers, most preferably wherein the polyvinylalcohol polymer comprises a blend of a

polyvinylalcohol homopolymer and a carboxylated anionic polyvinylalcohol copolymer or a blend of polyvinylalcohol homopolymers. Alternatively the water-soluble film may comprise a single polyvinylacohol polymer, preferably a carboxylated anionic polyvinylalcohol copolymer.

Preferred films exhibit good dissolution in cold water, meaning unheated distilled water. Preferably such films exhibit good dissolution at temperatures of 24°C, even more preferably at 10°C. By good dissolution it is meant that the film exhibits water-solubility of at least 50%, preferably at least 75% or even at least 95%, as measured by the method set out here after using a glass-filter with a maximum pore size of 20 microns, described above.

Preferred films are those supplied by Monosol under the trade references M8630, M8900, M8779, M8310.

The film may be opaque, transparent or translucent. The film may comprise a printed area. The area of print may be achieved using standard techniques, such as flexographic printing or inkjet printing.

The film may comprise an aversive agent, for example a bittering agent. Suitable bittering agents include, but are not limited to, naringin, sucrose octaacetate, quinine hydrochloride, denatonium benzoate, or mixtures thereof. Any suitable level of aversive agent may be used in the film. Suitable levels include, but are not limited to, 1 to 5000ppm, or even 100 to 2500ppm, or even 250 to 2000ppm.

Preferably, the water-soluble film or water-soluble unit dose article or both are coated in a lubricating agent, preferably, wherein the lubricating agent is selected from talc, zinc oxide, silicas, siloxanes, zeolites, silicic acid, alumina, sodium sulphate, potassium sulphate, calcium carbonate, magnesium carbonate, sodium citrate, sodium tripolyphosphate, potassium citrate, potassium tripolyphosphate, calcium stearate, zinc stearate, magnesium stearate, starch, modified starches, clay, kaolin, gypsum, cyclodextrins or mixtures thereof.

Detergent composition

The water-soluble unit dose article comprises a detergent composition, the composition can be any cleaning or treatment composition such as a hard surface cleaning composition, an automatic dishwashing cleaning composition, a laundry composition, etc. Preferably the composition is a laundry detergent composition. The term 'liquid laundry detergent composition' refers to any laundry detergent composition comprising a liquid capable of wetting and treating a fabric, and includes, but is not limited to, liquids, gels, pastes, dispersions and the like. The liquid composition can include solids or gases in suitably subdivided form, but the liquid composition excludes forms which are non-fluid overall, such as tablets or granules.

The laundry detergent composition can be used in a fabric hand wash operation or may be used in an automatic machine fabric wash operation.

The laundry detergent composition comprises a non-soap surfactant system and a metalloprotease. The non-soap surfactant system and the metalloprotease are described in more detail below.

The laundry detergent composition comprises from 30% to 65%, preferably from 30% to 55%, more preferably from 30% to 50% by weight of the composition of a non-soap surfactant system. The non-soap surfactant system comprises non-soap anionic surfactant and non-ionic surfactant. The anionic non-soap surfactant comprises from more than 0% and less than 20% by weight of the anionic non-soap surfactant of an anionic non-soap surfactant selected from the group consisting of alkyl sulphate, alkoxylated alkyl sulphate, and a mixture thereof.

Preferably, the weight ratio of non-soap anionic surfactant to non-ionic surfactant is greater than 1.5:1, preferably from greater than 1.5: 1 to 10: 1 or from 2: 1 to 5:1 or from 5:1 to 10: 1.

For surfactant weight % or weight ratio calculation the weight of the neutralizing counterion in the case of anionic surfactants is not taken into account, e.g. for soap or non-soap anionic surfactants solely the weight of the surfactant anion is considered when calculating the soap or non-soap anionic surfactant weight % or soap or non-soap anionic surfactant to nonionic surfactant weight ratio.

The non-soap anionic surfactant comprises an alkyl sulphate, an alkoxylated alkyl sulphate, or a mixture thereof. The (alkoxylated) alkyl sulphate anionic surfactant may be a primary or a secondary (alkoxylated) alkyl sulphate anionic surfactant, or a mixture thereof, preferably a primary (alkoxylated) alkyl sulphate anionic surfactant. Preferably, the alkoxylated alkyl sulphate comprises ethoxylated alkyl sulphate, propoxylated alkyl sulphate, a mixed ethoxylated/propoxylated alkyl sulphate, or a mixture thereof, more preferably an ethoxylated alkyl sulphate. Preferably, the ethoxylated alkyl sulphate has an average degree of ethoxylation of between 0.1 to 5, preferably between 0.5 and 3. Alternatively the alkyl sulphate anionic surfactant is free of alkoxylation. Preferably, the (alkoxylated) alkyl sulphate anionic surfactant has an average alkyl chain length of between 8 and 18, more preferably between 10 and 16, most preferably between 12 and 15. Preferably, the alkyl chain of the (alkoxylated) alkyl sulphate anionic surfactant is linear, branched, or a mixture thereof. Preferably, the branched (alkoxylated) alkyl sulphate anionic surfactant is a branched primary (alkoxylated) alkyl sulphate, a branched secondary (alkoxylated) alkyl sulphate, or a mixture thereof, preferably a branched primary (alkoxylated) alkyl sulphate, wherein the branching preferably is in the 2-position, or alternatively might be present further down the alkyl chain, or could be multi-branched with branches spread over the alkyl chain. The weight average degree of branching of (alkoxylated) alkyl sulphate anionic surfactant may be from 0% to 100% preferably from 0% to 95%, more preferably from

0% to 60%, most preferably from 0% to 20%. Alternatively, the weight average degree of branching of the (alkoxylated) alkyl sulphate anionic surfactant may be from 70% to 100%, preferably from 80% to 90%. Preferably, the alkyl chain is selected from naturally derived material, synthetically derived material or mixtures thereof. Preferably, the synthetically derived material comprises oxo-synthesized material, Ziegler-synthesized material, Guerbet-synthesized material, aldol condensation-synthesized material, Fischer-Tropsch - synthesized material, iso-alkyl synthesized material, or mixtures thereof, preferably oxo-synthesized material. Preferably, the laundry detergent composition comprises between 1% and 20%, preferably between 2% and 15%, more preferably between 4% and 10% by weight of the laundry detergent composition of the (alkoxylated) alkyl sulphate anionic surfactant. When alkyl ethoxy sulphate is present in the laundry detergent composition, the alkyl ethoxy sulphate starting material may have been treated to reduce the 1,4-dioxane content down to a level as low as less than 1 ppm per surfactant active. The skilled person will be aware of technical means to reduce the dioxane content in surfactant starting materials, including (multi-step) steam stripping, nano-filtration, or a combination thereof.

The non-soap anionic surfactant can comprise linear alkylbenzene sulphonate. Preferably, the linear alkylbenzene sulphonate comprises $C_{10}$-$C_{16}$ alkyl benzene sulfonate, $C_{11}$-$C_{14}$ alkyl benzene sulphonate or a mixture thereof. Preferably, the alkylbenzene sulphonate is an amine neutralized alkylbenzene sulphonate, an alkali metal neutralized alkylbenzene sulphonate or a mixture thereof. The amine is preferably selected from monoethanolamine, triethanolamine, monoisopropanolamine, or mixtures thereof, preferably the amine is monoethanol amine. The alkali metal is preferably selected from sodium, potassium, magnesium, or a mixture thereof. Preferably, the laundry detergent composition comprises between 5% and 45%, preferably between 7.5% and 40%, more preferably between 10% and 35% by weight of the laundry detergent composition of the linear alkylbenzene sulphonate.

Preferably the non-soap anionic surfactant comprises at least 80% by weight of the anionic non-soap surfactant of a linear alkylbenzene sulphonate anionic surfactant. Most preferably the anionic non-soap surfactant consists of linear alkyl benzene sulphonate anionic surfactant and alkyl sulphate, alkoxylated alkyl sulphate, or a mixture thereof. Preferably the anionic non-soap surfactant system consists of more than 0% and up to 20% of linear alkyl benzene sulphonate anionic surfactant and of at least 80% of alkyl sulphate anionic surfactant, alkoxylated alkyl sulphate anionic surfactant, or a mixture thereof.

The laundry detergent composition comprises a non-ionic surfactant. The non-ionic surfactant comprises, preferably consists of, an ethoxylated alcohol non-ionic surfactant. Preferably, the laundry detergent composition comprises between 5% and 45%, or between 10% and 40%, or between 15% and 35% by weight of the laundry detergent composition of the ethoxylated alcohol non-ionic surfactant. The ethoxylated alcohol non-ionic surfactant may be a primary nonionic surfactant, a secondary nonionic surfactant, or a mixture thereof. Preferably the nonionic surfactant comprises a mixture of primary and secondary ethoxylated alcohol nonionic surfactant, more preferably wherein the primary and the secondary ethoxylated alcohol nonionic surfactant are present in a weight ratio of from 2:1 to 1:10, preferably from 1.5:1 to 1:7, more preferably from 1:1 to 1:5. The ethoxylated alcohol nonionic surfactant may be linear or may be branched. When branched, the branching may be at the 1-position, the 2-position or even further down the alkyl chain, wherein the carbon counting starts as of the carbon linked to the oxygen linker between the alkyl chain and the ethoxylation chain, The branching may be a single branching or a multi-branching. Most preferably the branching is a single branching at the 2-position. The branching preferably is an alkyl branching, more preferably a methyl, ethyl, propyl, butyl or pentyl branching, most preferably mixtures thereof. When linear the alkyl chain of the alcohol may have a natural distribution of C6 to C20 alkyl chains pending the source of the material. Alternatively, the linear alkyl alcohol may have been fractionated to magnify the C12 to C14 alkyl chain content. The ethoxylated alcohol non-ionic surfactant comprises an alkyl chain having an average of from 8 to 18 carbon atoms, preferably of from 10 to 16 more preferably 12 to 15 carbon atoms. The ethoxylated alcohol nonionic surfactant has an average degree of ethoxylation between 5 and 12, preferably between 6 and 10. The ethoxylated alcohol nonionic surfactant may have a broad range (BRE) or a narrow range (NRE) ethoxylation distribution. Narrow-range ethoxylates (NREs) are alcohol polyglycol ethers with a narrow homolog distribution and are known nonionic surfactants. Peaked alkoxylation and peaked ethoxylation are also often used to describe the process and materials produced. They can be produced industrially, for example, by the addition of ethylene oxide onto alcohols in the presence of suitable catalysts (layer compounds which have been calcined or hydrophobized with fatty acids). Examples of narrow range alkoxylation catalysts include many alkaline earth (Mg, Ca, Ba, Sr, etc.) derived catalysts, Lewis acid catalysts, such as Zirconium dodecanoxide sulfate, and certain boron halide catalysts, such as those described by Dupont and of the form MB(OR1)x(X)4-x or B(OR1)3/ MX wherein R1 is a linear, branched, cyclic, or aromatic hydrocarbyl group, optionally substituted, having from 1 to 30 carbon atoms, M is Na+, K+, Li+, R2R3R4R5N+, or R2R3R4R5P+, where R2, R3, R4, and R5 independently are hydrocarbyl groups, and x is 1 to 3. This process can also be carried out on a variety of other hydrophobes and using different alkoxylating compounds (e.g., propylene oxide and butylene oxide) by modifying the catalyst properties. The narrow range ethoxylated alcohol non-ionic surfactant comprises at least 85% by weight of the total narrow range ethoxylate alcohol surfactant of alcohol ethoxylate nonionic surfactant molecules comprising a polyethoxy group comprising between 5 and 12, preferably between 6 and 10 ethoxy groups. The broad range ethoxylated alcohol non-ionic surfactant comprises polyethoxy groups, preferably, wherein between 15% and

45%, preferably between 25% and 40% by weight of the total broad range ethoxylated alcohol surfactant are ethoxylated alcohol nonionic surfactant molecules comprising a polyethoxy group comprising between 6 and 10 ethoxy groups, and wherein between 30% and 70%, preferably between 40% and 65% by weight of the total broad range ethoxylated alcohol surfactant are ethoxylated alcohol nonionic surfactant molecules comprise a polyethoxy group comprising between 5 and 12 ethoxy groups. The ethoxylated alcohol non-ionic surfactants may be derived from a natural alcohol source, a synthetic alcohol source, or a mixture thereof. Most suitable natural sources include palm kernel oil, coconut oil, or mixtures thereof, preferably palm kernel oil. When the ethoxylated alkyl alcohol non-ionic surfactant is derived from a synthetic alcohol source, the synthetic alcohol source preferably is made via an oxo process, a Ziegler process, a Guerbet process, an aldol condensation process, or a mixture thereof. The resulting alcohols can optionally but preferably be further fractionated to magnify the C12 to C15 content within the starting alcohol. Suitable examples of narrow range ethoxylated alcohol non-ionic surfactants are commercially available from the Nouryon company under the Berol or Ethylan tradenames, and from the Sasol company under the Novel tradename.

The laundry detergent composition may comprise a fatty acid, preferably a neutralized fatty acid soap. The fatty acid soap may be an amine neutralized fatty acid soap, wherein the amine is an alkanolamine more preferably selected from monoethanolamine, diethanolamine, triethanolamine, monoisopropanolamine, or a mixture thereof, more preferably monoethanolamine. The laundry detergent composition may comprise between 1.5% and 20%, preferably between 3% and 17%, more preferably between 5% and 15% by weight of the laundry detergent composition of fatty acid, preferably a neutralized fatty acid soap.

The laundry detergent comprises up to 15% by weight of the composition of water, preferably between 5% and 15%, more preferably between 7% and 15% by weight of the detergent composition of water.

Metalloprotease

The term "metalloprotease" is used herein in its conventional sense within the field of enzyme research and denotes a protease that requires a bound metal ion in order to be catalytically active. This is usually a zinc ion. The metalloproteases are preferably neutral metalloproteases, i.e. those that inter alia are active even at a neutral pH and require zinc ions for their catalytic effect. The molecular weight of proteases of this kind is usually in the range of from about 30 to about 40 kDa. The neutral metalloproteases are also referred to as "neutral metalloendopeptidases".

In various embodiments, at least one metalloprotease is selected from metalloproteases from B. amyloliquefaciens, Bacillus, Geobacillus, Alicyclobacillus, Lactobacillus, Exiguobacterium, Brevibacillus, Paenibacillus, Herpetosiphon, Oceanobacillus, Shewanella, Clostridium, Staphylococcus, Flavobacterium, Stigmatella, Myxococcus, Methanosarcina, Chryseobacterium, Streptomyces, Kribbella, Janibacter, Nocardioides, Xanthamonas, Micromonospora, Burkholderia, Dehalococcoides, Croceibacter, Kordia, Microscilla, Thermoactinomyces, Chloroflexus, Listeria, Plesiocystis, Haliscomenobacter, Cytophaga, Hahella, Arthrobacter, Brachybacterium, Clavibacter, Microbacterium, Intrasporangium, Frankia, Meiothermus, Pseudomonas, Ricinus, Catenulispora, Anabaena, Nostoc, Halomonas, Chromohalobacter, Bordetella, Variovorax, Dickeya, Pectobacterium, Citrobacter, Enterobacter, Salmonella, Erwinia, Pantoea, Rahnella, Geodermatophilus, Gemmata, Xenorhabdus, Photorhabdus, Aspergillus, Neosartorya, Pyrenophora, Saccharopolyspora, Nectria, Gibberella, Metarhizium, Waddlia, Cyanothece, Cellulphaga, Providencia, Bradyrhizobium, Agrobacterium, Mucilaginibacter, Serratia, Sorangium, Streptosporangium, Renibacterium, Aeromonas, Chromobacterium, Moritella, Haliangium, Kangiella, Marinomonas, Vibrionales, Listonella, Salinivibrio, Photobacterium, Alteromonadales, Legionella, Teredinibacter, Reinekea, Hydrogenivirga, Pseudoalteromonas, Kribella, Thermoascus, Lysobacter, Achromobacter, Aeromonas and Streptomyces.

Examples of such metalloproteases and their variants include those described in: WO2015/158723 (SEQ ID NO:2), WO2015/193488 (SEQ ID NO:2) , WO2014/029819 (SEQ ID NO:2), WO2014/029820 (SEQ ID NO:2 and SEQ ID NO:4), WO2016/075078 (SEQ ID NO:2 and SEQ ID NO:4), WO2014/029821 (SEQ ID NO:2 and SEQ ID NO:4), WO2019/105675 (SEQ ID NO: 1), WO2019/142774, JP7057140 B2 (SEQ ID NO: 2, 4, 6, 8).

Preferably the protease is selected from the M4, M5, M7, M8, M23 or M35 family, more preferably an M4 metalloprotease, most preferably a neutral metalloprotease.

The term "M4 Metalloprotease Family" or "M4 Metalloprotease" or "M4" as used herein means a polypeptide falling into the M4 metalloprotease family according to Rawlings et al., Biochem. J., 290, 205-218 (1993) and as further described in MEROPS - (Rawlings et al., MEROPS: the peptidase database, Nucl Acids Res, 34 Database issue, D270-272, 2006). The M4 metalloproteases are neutral metalloproteases containing mainly endopeptidases. All peptidases in the family bind a single, catalytic zinc ion. M4 metalloprotease family members include the common HEXXH motif, where the histidine residues serve as zinc ligands and glutamate is an active site residue. M4 metalloproteases have a pH optimum mainly at neutral pH. The M4 metalloprotease family includes, e.g., Neutrase™ (Novozymes) (classified as MEROPS subclass M04.014), Thermolysin, Bacillolysin, vibriolysin, pseudolysin, Msp peptidase, coccolysin, aureolysin, vimelysin, lambda toxin neutral peptidase B, PA peptidase (Aeromonas- type), griselysin, stearolysin, MprIII (Alteromonas sp. strain 0-7), pap6 peptidase, neutral peptidase (Thermoactinomyces-type), ZmpA peptidase (Burkholderia sp.), zpx

peptidase, PrtS peptidase (Photorhabdus luminescens), protealysin, ZmpB peptidase (Burkholderia sp.). The M4 metalloprotease family of polypeptides have been further characterized and presently includes, according to MEROPS, at least twenty-two subclasses for which a distinct MEROPS ID (i.e., an identifier of the formula M04.xxx) has been assigned, as well as non-peptidase homologues and unassigned peptidases.

The term "M5 Metalloprotease Family" or "M5 Metalloprotease" or "M5" or "mycolysin family" as used herein means a polypeptide falling into the M5 metalloprotease family according to Rawlings et al., Biochem. J., 290, 205-218 (1993) and as further described in MEROPS - (Rawlings et al., MEROPS: the peptidase database, Nucl Acids Res, 34 Database issue, D270- 272, 2006). The term "M7 Metalloprotease Family" or "M7 Metalloprotease" or "M7" or "snapalysin family" as used herein means a polypeptide falling into the M7 metalloprotease family according to Rawlings et al., Biochem. J., 290, 205-218 (1993) and as further described in MEROPS - (Rawlings et al., MEROPS: the peptidase database, Nucl Acids Res, 34 Database issue, D270- 272, 2006). The protease family M7 contains a metalloendopeptidase, snapalysin.

The term "M8 Metalloprotease Family" or "M8 Metalloprotease" or "M8" or "leishmanolysin family" as used herein means a polypeptide falling into the M8 metalloprotease family according to Rawlings et al., Biochem. J., 290, 205-218 (1993) and as further described in MEROPS - (Rawlings et al., MEROPS: the peptidase database, Nucl Acids Res, 34 Database issue, D270- 272, 2006).

The term "M23 Metalloprotease Family" as used herein means a polypeptide falling into the M23 metalloprotease family according to Proteolysis in Cell Function, pp13-21 , IOS Press, Amsterdam (1997), Rawlings et al., Biochem. J., 290, 205-218 (1993) and as further described in MEROPS - (Rawlings et al., MEROPS: the peptidase database, Nucl Acids Res, 34 Database issue, D270- 272, 2006). The M23 family includes stapholysin and lysostaphin.

The term "M35 Metalloprotease Family" or "M 35 Metalloprotease" or "M 35" or "deuterolysin family" as used herein means a polypeptide falling into the M35metalloprotease family according to Proteolysis in Cell Function, pp13-21 , IOS Press, Amsterdam (1997), Rawlings et al., Biochem. J., 290, 205-218 (1993) and as further described in MEROPS - (Rawlings et al., MEROPS: the peptidase database, Nucl Acids Res, 34 Database issue, D270- 272, 2006).

Preferred metalloproteases include thermolysin, matrix metalloproteinases and those metalloproteases derived from Bacillus subtilis, Bacillus thermoproteolyticus, Geobacillus stearothermophilus or Geobacillus sp., or Bacillus amyloliquefaciens, as described in US PA 2008/0293610A1, or Paenibacillus hunanensis. A specially preferred metalloprotease belongs to the M4 family according to Rawlings et al., Biochem. J., 290, 205-218 (1993) and as further described in MEROPS - (Rawlings et al., MEROPS: the peptidase database, Nucl Acids Res, 34 Database issue, D270-272, 2006). Specially preferred for use herein are metalloproteases derived from Paenibacillus hunanensis.

In one aspect, suitable metalloproteases are the neutral metalloprotease variants described in WO2007/044993, WO2009/058661 and US 20140315775. In one aspect the protease is a variant having at least 80%, or 85% or 90% or 95% or 96% or 97% or 98% or 99% or even 100% identity to SEQ ID NO:18 of WO2007/044993, including those with substitutions at one or more of the following positions versus SEQ ID NO:18 of WO2007/044993: T004C, T004E, T004H, T004I, T004K, T004L, T004M, T004N, T004P, T004R, T004S, T004V, T004W, T004Y, G012D, G012E, G012I, G012K, G012L, G012M, G012Q, G012R, G012T, G012V, G012W, K013A, K013C, K013D, K013E, K013F, K013G, K013H, K013I, K013L, K013M, K013N, K013Q, K013S, K013T, K013V, K013Y,T014F, T014G, T014H, T014I, T014KJ014L, T014M, T014P, T014Q, T014R, T014S, T014V, T014W, T014Y, S023A, S023D, S023F, S023G, S023I, S023K, S023L, S023M, S023N, S023P, S023Q, S023R, S023S, S023T, S023V, S023W, S023Y, G024A, G024D, G024F, G024G, G024H, G024I, G024K, G024L, G024M, G024N, G024P, G024R, G024S, G024T, G024V, G024W, G024Y, K033H, Q045C, Q045D, Q045E, Q045F, Q045H, Q045I, Q045K, Q045L, Q045M, Q045N, Q045P, Q045R, Q045T, Q045W, N046A, N046C, N046E, N046F, N046G, N046H, N046I, N046K, N046L, N046M, N046P, N046Q, N046R, N046S, N046T, N046V, N046W, N046Y, R047E, R047K, R047L, R047M, R47Q, R047S, R047T, Y049A, Y049C, Y049D, Y049E, Y049F, Y049H, Y049I, Y049K, Y049L, Y049N, Y049R, Y049S, Y049T, Y049V, Y049W, N050D, N050F, N050G, N050H, N050I, N050K, N050L, N050M, N050P, N050Q, N050R, N050W, N050Y, T054C, T054D, T054E, T054F, T054G, T054H, T054I T054K, T054L, T054M, T054N, T054P, T054Q, T054R, T054S, T054V, T054W, T054Y, S058D, S058H, S058I, S058L, S058N, S058P, S058Q, T059A, T059C, T059E, T059G, T059H, T059I, T059K, T059L T059M, T059N, T059P, T059Q, T059R, T059S, T059V,T059W, T060D, T060F, T060I, T060K, T060L, T060N, T060Q, T060R, T060V, T060W, T060Y, T065C, T065E,T065F, T065H, T065I, T065K, T065L, T065M, T065P, T065Q, T065R, T065V, T065Y, S066C, S066D, S066E, S066F, S066H, S066I, S066K, S066L,S066N, S066P, S066Q, S066R, S066T, S066V, S066W, S066Y, Q087A, Q087D, Q087E, Q087H, Q087I, Q087K, Q087L, Q087M, Q087N, Q087R, Q087S, Q087T, Q087V, Q087W, N090C, N090D, N090E, N090F, N090G, N090H, N090K, N090L, N090R, N090T, N096G, N096H, N096K, N096R, K097H, K097Q, K097W, K100A, K100D, K100E1 K100F, K100H, K100N, K100P, K100Q, K100R, K100S, K100V, K100Y, R110A, R110C, R110E, R110H, R110K, R110L, R110M, R110N, R110Q, R110S, R110Y, D119E, D119H, D119I, D119L, D119Q, D119R, D119S, D119T, D119V, D119W, G128C, G128F, G128H, G128K, G128L, G128M, G128N, G128Q, G128R, G128W, G128Y, S129A, S129C, S129D, S129F, S129G, S129H, S129I, S129K, S129L, S129M, S129Q, S129R, S129T, S129V, S129W, S129Y, F130I, F130K, F130L, F130M, F130Q, F130R, F130T, F130V, F130Y, S135P, G136I, G136L, G136P, G136V, G136W, G136Y, S137A,M138I, M138K, M138L, M138Q, M138V, D139A, D139C, D139E, D139G, D139H, D139I,

D139K, D139L, D139M, D139P, D139R, D139S, D139V, D139W, D139Y, V140C, Q151I, E152A, E152C, E152D, E152F, E152G, E152H, E152L, E152M, E152N, E152R, E152S, E152W, N155D, N155K, N155Q, N155R, D178A, D178C, D178G, D178H, D178K, D178L, D178M, D178N, D178P, D178Q, D178R, D178S, D178T, D178V, D178W, D178Y, T179A,T179F, T179H, T179I, T179K, T179L, T179M, T179N, T179P, T179Q, T179R, T179S, T179V, T179W, T179Y, E186A, E186C, E186D, E186G, E186H, E186K, E186L, E186M, E186N, E186P, E186Q, E186R, E186S, E186T, E186V, E186W, E186Y, V190H, V190I, V190K, V190L, V190Q, V190R, S191F, S191G, S191H, S191I, S191K, S191L, S191N, S191Q, S191R, S191W, L198M, L198V, S199C, S199D, S199E, S199F, S199I, S199K, S199L, S199N, S199Q, S199R, S 199V, Y204H, Y204T, G205F, G205H, G205L, G205M, G205N, G205R, G205S, G205Y, K211A, K211C, K211D, K211G, K211M, K211N, K211Q, K211R, K211S, K211T, K211V, K214A, K214C, K214E, K214I, K214L, K214M, K214N, K214Q, K214R, K214S, K214V, L216A, L216C, L216F, L216H, L216Q, L216R, L216S, L216Y, N218K, N218P, T219D, D220A, D220E, D220H, D220K, D220N, D220P, A221D, A221E, A221F, A221I,A221K, A221L, A221M, A221N, A221S, A221V, A221Y, G222C, G222H, G222N, G222R, Y224F, Y224H, Y224N, Y224R, T243C, T243G, T243H, T243I, T243K, T243L, T243Q, T243R, T243W, T243Y, K244A, K244C, K244D, K244E, K244F, K244G, K244L, K244M, K244N, K244Q, K244S, K244T, K244V, K244W, K244Y, V260A, V260D, V260E, V260G, V260H, V260I, V260K, V260L,V260M, V260P, V260Q, V260R V260S, V260T, V260W,V260Y, Y261C, Y261F, Y261I, Y261L, T263E, T263F, T263H, T263I, T263L, T263M, T263Q, T263V, T263W, T263Y, S265A, S265C, S265D, S265E, S265K, S265N, S265P, S265Q, S265R,S265T, S265V, S265W, K269E, K269F, K269G, K269H, K269I, K269L, K269M, K269N, K269P, K269Q, K269S, K269T, K269V, K269W, K269Y, A273C, A273D, A273H, A273I, A273K, A273L, A273N, A273Q, A273R, A273Y, R280A, R280C, R280D, R280E, R280F, R280G, R280H, R280K, R280L, R280M, R280S, R280T, R280V, R280W, R280Y, L282F, L282G, L282H, L282I, L282K, L282M, L282N, L282Q, L282R, L282V, L282Y, S285A, S285C, S285D, S285E, S285K, S285P, S285Q, S285R, S285W, Q286A, Q286D, Q286E, Q286K, Q286P, Q286R, A289C, A289D, A289E, A289K, A289L, A289R, A293C, A293R, N296C, N296D, N296E, N296K, N296R, N296V, A297C, A297K, A297N, A297Q, A297R and G299N.

In some preferred embodiments, the metalloprotease is a metalloprotease variant having a sequence identity of at least 80%, or 85% or 90% or 95% or 96% or 97% or 98% or 99% or even 100% identity to SEQ ID NO:18 of WO2007/044993, including those with substitutions at one or more of the following positions SEQ ID NO:18 of WO2007/044993: S129I, S129V, S129L, F130L, M138I, M138L, V190I, V190L, D220E and D220P, particularly preferred S129I, F130L, M138L, V190I, D220P (SEQ ID NO:20 in WO2009/058661).

Further suitable metalloproteases are derived from a member of the order Bacillales; family Bacillaceae, Paenibacillaceae, Geobacillus, Brevibacillus, Paenibacillus, Alicyclobacillaceae, Lactobacillaceae, Exiguobacterium spp. as described in WO2014194034 and WO2014194117. In one aspect the protease is derived from Paenibacillus hunanensis described in WO2014194034. In one aspect the protease is having at least 80%, or 85% or 90% or 95% or 96% or 97% or 98% or 99% or even 100% identity to SEQ ID NO: 33 of WO2014194034.

The neutral metalloproteases of the present invention are also referred to as "neutral metalloendopeptidases" and include the enzymes in class EC 3.4.24.

Suitable commercially available metalloprotease enzymes include those sold under the trade names Neutrase® by Novozymes A/S (Denmark), the Corolase® range including Corolase® 8000 and Corolase® 7089 from AB Enzymes, Protex 14L and Protex 15L from IFF (Palo Alto, Calif.), those sold as thermolysin from Sigma and the Thermoase range (PC10F and C100) and thermolysin enzyme from Amano enzymes.

Preferred levels of metalloprotease in the product of the invention include from about 0.05 to about 10 mg, more preferably from about 0.5 to about 7 mg and especially from about 1 to about 6 mg of active metalloprotease/g of composition.

Proteases

The composition of the invention can comprise one or more proteases, preferably the composition of the invention comprises at least one protease. A mixture of two or more proteases can contribute to an enhanced cleaning across a broader temperature, cycle duration, and/or substrate range, and provide superior cleaning benefits.

Suitable proteases for use in combination with the metalloprotease are serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

i) subtilisins (EC 3.4.21.62), especially those derived from Bacillus, such as Bacillus sp., B. lentus, B. alkalophilus, B. subtilis, B. amyloliquefaciens, B. gibsonii, B. akibaii, Bacillus Clausii and B. clarkii described in WO2004067737, WO2015091989, WO2015091990, WO2015024739, WO2015143360, US 6,312,936 B1, US 5,679,630, US 4,760,025, WO03/055974, WO03/054185, WO03/054184, WO2017/215925, DE102006022216,

WO2015089447, WO2015089441A1, WO2016066756, WO2016066757, WO2016069557, WO2016069563, WO2016069569, WO2016174234, WO2017/089093, WO2020/156419, WO2016/183509. Specifically, mutations S9R, A15T, V66A, A188P, V199I, N212D, Q239R, N255D, X9E, X200L, X256E, X9R, X19L, X60D (Savinase numbering system)

ii) subtilisins from B. Pumilus such as the ones described in DE102006022224A1, WO2020/221578, WO2020/221579, WO2020/221580, including variants comprising amino acid substitutions in at least one or more of the positions selected from 9, 130, 133, 144, 224, 252, 271 (BPN' numbering system).

iii) trypsin-type or chymotrypsin-type proteases, such as trypsin (e.g., of porcine or bovine origin), including the Fusarium protease described in WO 89/06270 and the chymotrypsin

iv) proteases derived from Cellumonas described in WO 05/052161 and WO 05/052146

v) proteases having at least 90% identity to the subtilase from Bacillus sp. TY145, NCIMB 40339, described in WO92/17577 (Novozymes A/S), including the variants of this Bacillus sp TY145 subtilase described in WO2015024739, and WO2016066757.

Preferred additional proteases for the detergent of the invention are polypeptides demonstrating at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Especially preferred additional protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).

(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD
(viii) S9R+A15T+V68A+N218D+Q245R

The most preferred additional protease is a subtilase variant derived from B. amyloliquefaciens (BPN'), as described in WO2011/072117. Especially useful BPN' variant comprises mutation in one or more of the following positions: X003Q, X006W, X022Y, X024K, X024Q, X024G, X033T, X045V, X053G, X055P, S063T, X076D, X078N, X087D, X101N, X109Q, X118R, X128A, X128S, X145R, X166Q, X169A, X162Q, X182Q, X183N, S183T, X204Q, X206Y, X217Q, Y217L, X218S, X222Q, X248A or X254A; (in BPN' numbering system).

Suitable commercially available additional protease enzymes include:

i) those sold under the trade names Alcalase®, Savinase®, Primase®, Durazym®, Polarzyme®, Kannase®, Liquanase®, Liquanase Ultra®, Savinase Ultra®, Liquanase® Evity®, Savinase® Evity®, Ovozyme®, Neutrase®, Everlase®, Coronase®, Blaze®, Blaze Ultra®, Blaze® Evity®, Blaze® Exceed, Blaze® Pro, Esperase®, Progress® Uno, Progress® Excel, Progress® Key, Ronozyme®, Vinzon® and Het Ultra® by Novozymes A/S (Denmark);

those sold under the tradename Maxatase®, Maxacal®, Maxapem®, Properase®, Purafect®, Purafect Prime®, Purafect Ox®, FN3®, FN4®, Excellase®, Ultimase® and Purafect OXP®, Preferenz® by IFF (formerly Dupont);

ii) those sold under the tradename Opticlean® and Optimase® by Solvay Enzymes;

those available from Henkel/Kemira, namely BLAP (sequence shown in Figure29 of US 5,352,604 with the following mutations S99D + S101R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and can comprise a further mutation 101E/D, S156D, L262E, 206A/L/S/T/, 209K/V/W, 215W, 216N/S/T; and

iii) those sold under the tradename and Lavergy®, Lavergy® Pro, Lavergy® C Bright from BASF.

Especially preferred for use herein in combination with the metalloprotease of the invention are commercial proteases selected from the group consisting of Properase®, Blazed, Ultimase®, Everlase®, Savinase®, Excellase®, Blaze Ultra®, BLAP and BLAP variants (Lavergy® Pro).

Preferred levels of protease in the product of the invention include from about 0.05 to about 10 mg, more preferably from about 0.5 to about 7 mg and especially from about 1 to about 6 mg of active protease/g of composition.

Preferably, the laundry detergent composition comprises between 5% and 30%, preferably between 10% and 30% by weight of the laundry detergent composition of a non-aqueous organic solvent, preferably wherein the non-aqueous organic solvent is selected from 1,2-propanediol, dipropylene glycol, tripropyleneglycol, glycerol, sorbitol, polyethylene glycol, ethoxylated glycerin or a mixture thereof. Preferably the non-aqueous organic solvent comprises 1,2-propanediol and glycerol, more preferably wherein the 1,2-propanediol and glycerol are in a weight ratio of from 1:3 to 12: 1, preferably of from 1:2 to 9:1, more preferably from 1: 1 to 6:1. The laundry composition preferably comprises less than 1% preferably less than 0.5% by weight of the composition of ethanol, most preferably the laundry composition is free of ethanol.

Preferably, the laundry detergent composition comprises an adjunct ingredient selected from the group comprising builders, perfumes, enzymes, citrate, bleach, bleach catalyst, dye, hueing dye, brightener, cleaning polymers including alkoxylated polyamines and polyethyleneimines, soil release polymer, fabric care polymers including cationic hydroxyethyl celluloses, cationic guar gums and cationic polyglucans, surfactant, solvent, dye transfer inhibitors, chelant, encapsulated perfume, polycarboxylates, structurant, pH trimming agents, anti-oxidants including Ralox 35, anti-foam agent, and mixtures thereof.

Preferably, the laundry detergent composition comprises a further enzyme selected from the group comprising hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, ß-glucanases, arabinosidases, hyaluronidase, chondroitinase, laccase, xyloglucanases, mannanases and amylases, nuclease, pectate lyases, or mixtures thereof, preferably a further enzyme selected from the group comprising proteases, amylase, cellulase, lipases, xyloglucanases, mannanases, nucleases, pectate lyases, and mixtures thereof. Most preferably the laundry detergent composition comprises a protease. The detergent article preferably comprises at least two compartments, wherein the metalloprotease is present in a first compartment and another enzyme is present in another compartment.

Preferably, the laundry detergent composition has a pH between 6 and 10, more preferably between 6.5 and 8.9, most preferably between 7 and 8, wherein the pH of the laundry detergent composition is measured as a 10% product concentration in demineralized water at 20°C.

When liquid, the liquid laundry detergent composition may be Newtonian or non-Newtonian. Preferably, the liquid laundry detergent composition is non-Newtonian. Without wishing to be bound by theory, a non-Newtonian liquid has properties that differ from those of a Newtonian liquid, more specifically, the viscosity of non-Newtonian liquids is dependent on shear rate, while a Newtonian liquid has a constant viscosity independent of the applied shear rate. The decreased viscosity upon shear application for non-Newtonian liquids is thought to further facilitate liquid detergent dissolution. The liquid laundry detergent composition described herein can have any suitable viscosity depending on factors such as formulated ingredients and purpose of the composition.

Process of making

Those skilled in the art will be aware of standard techniques to make the laundry detergent composition and the water-soluble unit dose article according to the present invention. Those skilled in the art will also be aware of standard techniques and methods to make the ingredients of the laundry detergent composition of the present invention.

Process of use

A further aspect of the present invention is a process of laundering fabrics comprising the steps of diluting between 200 and 3000 fold, preferably between 300 and 2000 fold, the water-soluble unit dose article according to the present invention with water to make a wash liquor, contacting fabrics to be treated with the wash liquor.

Preferably the wash liquor comprises between 5L and 75L, preferably between 7L and 40L, more preferably between 10L and 20L of water. Alternatively, the wash liquor may comprise between 35L and 65L of water. Preferably, the wash liquor is at a temperature of between 5°C and 90°C, preferably between 10°C and 60°C, more preferably between 12°C and 45°C, most preferably between 15°C and 40°C. Preferably, washing the fabrics in the wash liquor takes between 5 minutes and 60 minutes, preferably between 5 minutes and 40 minutes, more preferably between 5 minutes and 30 minutes, even more preferably between 5 minutes and 20 minutes, most preferably between 6 minutes and 18 minutes to complete. Alternatively, washing the fabrics in the wash liquor may take between 30 minutes and 60 minutes. Preferably, the wash liquor comprises between 1kg and 20 kg, preferably between 3kg and 15kg, most preferably between 5 and 10 kg of fabrics. The wash liquor may comprise water of any hardness preferably varying between 0 gpg to 40gpg.

The dimensions and values disclosed herein are not to be understood as being strictly limited to the exact numerical values recited. Instead, unless otherwise specified, each such dimension is intended to mean both the recited

value and a functionally equivalent range surrounding that value. For example, a dimension disclosed as "40 mm" is intended to mean "about 40 mm."

EXAMPLES

The impact of metalloprotease on stain removal varying the level of alkyl ethoxy sulphate (AES) surfactant was tested.

**Wash test procedure using an automated tergotometer**

1. 1 litre of 21 US grains per gallon (gpg) hard water was heated to 30°C and added to each tergotometer pot.
2. 1.7g of the detergent were added to the tergotometer wash pots, followed by the spike of the metalloprotease enzyme, as shown in Table 1.
3. To ensure detergent was thoroughly mixed with wash water, the tergotometer mixed the wash water for 1 minute at 417 RPM.
4. 10 swatches (5x5cm) of CFT stains were added to the tergotometer pot (2 swatches per each stain type: Blood, Milk, Ink, on woven cotton (C-05), Pigment, Oil, Milk (POM), on woven cotton (C-10), Full Egg with Carbon Black (CB), aged, on woven cotton (C-S-39), Grass, on woven cotton (C-S-07), Chocolate soymilk drink (CSM), aged, on woven cotton (C-S-45), Blood, sheep's, double application, aged, on woven cotton (C-S-85)), followed by the addition of 10 pieces 6x6cm SBL 2004 sheets (supplied by WFK - Testgewebe GmbH, Brüggen-Bracht, Germany ), and followed by addition of 5x5cm knitted cotton ballast (supplied by Warwick Equest, Consett, United Kingdom), to give a total load weight of 60 g fabrics.
5. The tergotometer was set to mix at 208 RPM for 30 minutes to simulate a main wash process.
6. Wash water was then removed and replaced with 1 liter of 21 grains per gallon (gpg) water at 30°C.
7. The tergotometer was set to mix at 208 RPM for 5 minutes to simulate a rinse process.
8. Wash water was again removed and replaced a second time with 1 litre of 21 grains per gallon (gpg) water at 30°C.
9. The tergotometer was set to mix at 208 RPM for 5 minutes to simulate the second rinse process.
10. All fabrics were then removed from each pot.
11. Steps 1-10 were repeated a further 3 times.
12. CFT stained tracers were separated from wash ballast, tumble dried and packed in aluminum foil to protect from light and humidity before next day evaluation.
13. The fabrics were analysed using image analysis software for L*a*b* values for unwashed stains, washed stains and unsoiled fabric. Delta E* calculations were made to determine the level of staining for both unwashed stains and washed stains compared to the unsoiled fabric using the following equation where the suffix 1 denotes the values for the unsoiled fabric and the suffix 2 denotes the values for the unwashed or washed stains.

$$\Delta E^*_{AB} = \sqrt{(L^*_2 - L^*_1)^2 + (a^*_2 - a^*_1)^2 + (b^*_2 - b^*_1)^2}$$

The Stain Removal Index (SRI) is the level of stain removal calculated as a percentage as follows:

$$\mathrm{SRI} = 100 \times (\mathrm{A} - \mathrm{B}) / \mathrm{A}$$

Where:

A = Delta E* of Unwashed fabric stained region
B = Delta E* of Washed fabric stained region.

Table 2 shows the stain removal results.

Test formulations:

Table 1 shows the two nil metalloprotease reference formulations (examples A and B), varying in relative alkyl ethoxy sulfate (AES) content, as well as the related metalloprotease comprising test formulations (examples C and 1). All four formulations also comprise a constant level of a traditional protease.

Table 1: Test formulations

| Wt% - 100% active basis | Ex A* (High AES) | Ex B* (Low AES) | Ex C* (High AES) | Ex 1 (Low AES) |
|---|---|---|---|---|
| Water | 13.02 | 12.96 | 13.02 | 12.96 |
| Citric Acid | 0.69 | 0.69 | 0.69 | 0.69 |
| 1,2 Propylene Glycol | 12.36 | 23.13 | 12.36 | 23.13 |
| MEA | 10.44 | 9.43 | 10.44 | 9.43 |
| Glycerin | 5.03 | 5.03 | 5.03 | 5.03 |
| HEDP | 2.21 | 2.21 | 2.21 | 2.21 |
| $K_2SO_3$ | 0.40 | 0.40 | 0.40 | 0.40 |
| C12-14 AE7 | 3.42 | 3.42 | 3.42 | 3.42 |
| HLAS | 26.79 | 26.79 | 26.79 | 26.79 |
| C1214- $AE_3S$ | 10.71 | 3.23 | 10.71 | 3.23 |
| Brightener 49 | 0.30 | 0.30 | 0.30 | 0.30 |
| C12-18 TPK Fatty Acid | 5.31 | 3.09 | 5.31 | 3.09 |
| Zwitterionic polyamine[1] | 1.84 | 1.84 | 1.84 | 1.84 |
| Ethoxylated polyethyleneimine[2] | 1.57 | 1.57 | 1.57 | 1.57 |
| Amphiphilic graft copolymer[3] | 2.58 | 2.58 | 2.58 | 2.58 |
| $MgCl_2$ solution | 0.32 | 0.32 | 0.32 | 0.32 |
| nuclease | 0.012 | 0.012 | 0.012 | 0.012 |
| Amylase | 0.011 | 0.011 | 0.011 | 0.011 |
| Mannanase | 0.004 | 0.004 | 0.004 | 0.004 |
| Green Dye | 0.0075 | 0.0075 | 0.0075 | 0.0075 |
| Blue Dye | 0.0014 | 0.0014 | 0.0014 | 0.0014 |
| Yellow Dye | 0.0016 | 0.0016 | 0.0016 | 0.0016 |
| Perfume | 2.57 | 2.57 | 2.57 | 2.57 |
| Antifoam 8017 | 0.25 | 0.25 | 0.25 | 0.25 |
| Hydrogenated Castor Oil | 0.09 | 0.09 | 0.09 | 0.09 |
| Protease[4] | 0.07 | 0.07 | 0.07 | 0.07 |
| | | | | |
| Metalloprotease[5] | - | - | 1.11 ppm | 1.11 ppm |

(continued)

| Wt% - 100% active basis | Ex A* (High AES) | Ex B* (Low AES) | Ex C* (High AES) | Ex 1 (Low AES) |
|---|---|---|---|---|
| pH (10% solution in demineralized water) | 7.40 | 7.40 | 7.40 | 7.40 |

*comparative

[1] Lutensit Z96 (zwitterionic polyamine ex BASF - zwitterionic hexamethylene diamine according to below formula : 100% quaternized and about 40% of the polyethoxy (EO24) groups are sulfonated).

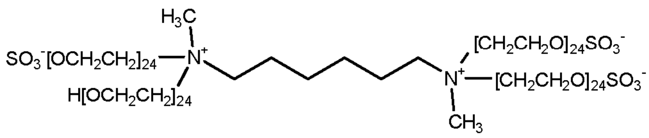

[2] Lutensol FP620 ex BASF - ethoxylated polyethyleneimine (PEI600 EO20)

[3] polyethylene glycol graft polymer comprising a polyethylene glycol backbone (Pluriol E6000) and hydrophobic vinyl acetate side chains, comprising 40% by weight of the polymer system of a polyethylene glycol backbone polymer and 60% by weight of the polymer system of the grafted vinyl acetate side chains

[4] A subtilase variant derived from B. amyloliquefaciens (BPN'), as described in WO2011/072117. Especially useful BPN' variant comprises mutation in one or more of the following positions: X003Q, X006W, X022Y, X024K, X024Q, X024G, X033T, X045V, X053G, X055P, S063T, X076D, X078N, X087D, X101N, X109Q, X118R, X128A, X128S, X145R, X166Q, X169A, X162Q, X182Q, X183N, S183T, X204Q, X206Y, X217Q, Y217L, X218S, X222Q, X248A or X254A; (in BPN' numbering system).

[5] SEQ ID NO:20 in WO2009/058661 - level = active protein level in wash solution, metalloprotease was spiked separately from remainder of detergent composition to the wash solution

Test results:

Table 2 summarizes the average stain removal performance for the four different test legs across a set of protease sensitive stains. The results illustrates that the performance impact of metalloprotease addition is higher at a low relative AES content according to the invention (index 145) when compared to the metalloprotease addition at a high relative AES content outside the scope of the invention (index 130).

Table 2: Impact of metalloprotease addition.

| %SRI | Average | Metalloprotease addition Index |
|---|---|---|
| Ex A | 29.2 | 100 |
| Ex B | 25.7 | 100 |
| Ex C | 38.0 | 130 |
| Ex 1 | 37.2 | 145 |

## Claims

**1.** A water-soluble unit dose detergent article comprising a water-soluble film and a laundry liquid detergent composition, wherein the detergent composition comprises:

a non-soap surfactant system comprising anionic non-soap surfactant and nonionic surfactant wherein the anionic non-soap surfactant comprises more than 0% and less than 20% by weight of the anionic non-soap surfactant of an anionic non-soap surfactant selected from the group consisting of alkyl sulphate, alkoxylated alkyl sulphate, and a mixture thereof;
a metalloprotease; and
up to 15% by weight of the composition of water.

2. The detergent article according to claim 1, wherein the anionic non-soap surfactant comprises linear alkyl benzene sulphonate.

3. The detergent article according to the preceding claim wherein the anionic non-soap surfactant comprises at least 80% by weight of the anionic non-soap surfactant of linear alkyl benzene sulphonate.

4. The detergent article according to any of the preceding claims, wherein the detergent composition comprises from 30% to 65% by weight of the composition of the non-soap surfactant system.

5. The detergent article according to any of the preceding claims, wherein the nonionic surfactant is selected from the group consisting of primary alcohol ethoxylate nonionic surfactant, secondary alcohol ethoxylate nonionic surfactant, and mixtures thereof.

6. The detergent article according to any of the preceding claims, wherein the detergent composition comprises from 5% to 30% by weight of the composition of organic solvent and wherein the organic solvent is preferably selected from the group consisting of: 1,2-propanediol, dipropylene glycol, tripropyleneglycol, glycerol, sorbitol, polyethylene glycol, ethoxylated glycerine, and a mixture thereof.

7. The detergent article according to any of the preceding claims, wherein the weight ratio of non-soap anionic surfactant to non-ionic surfactant is from greater than 1.5:1 to 10:1, preferably from 2:1 to 5:1.

8. The detergent article according to any of the preceding claims, wherein the detergent composition further comprises a fatty acid, preferably the detergent composition comprises between 1.5% and 20% by weight of the composition of the fatty acid.

9. The detergent article according to any of the preceding claims, wherein the metalloprotease is a metalloprotease selected from the M4, M7, M23 or M35 family, preferably a metalloprotease of the M4 family.

10. The detergent article according to any of the preceding claims, wherein the metalloprotease is a metalloprotease variant having a sequence identity of at least 80%, preferably at least 90%, preferably at least 95%, preferably at least 98% and more preferably at least 99% to the metalloprotease of SEQ ID NO:18 of WO2007/044993.

11. The detergent article according to the any of claims 1 to 9, wherein the metalloprotease is a metalloprotease variant having a sequence identity of at least 80%, preferably at least 90%, preferably at least 95%, preferably at least 98% and more preferably at least 99% to the metalloprotease of SEQ ID NO:20 of WO2009/058661 or to the metalloprotease of SEQ ID NO:33 of WO2014/194034.

12. The detergent article according to claim 10, wherein the metalloprotease is a metalloprotease variant having a sequence identity of at least 80%, preferably at least 90%, preferably at least 95%, preferably at least 98% and more preferably at least 99% to the metalloprotease of SEQ ID NO:18 of WO2007/044993 with substitutions at one or more of the following positions versus SEQ ID NO:18 of WO2007/044993: S129I, S129V, S129L, F130L, M138I, M138L, V190I, V190L, D220E and D220P, preferably with substitutions at one or more of the following positions versus SEQ ID NO: 18 of WO2007/044993: S129I, F130L, M138L, V190I and D220P.

13. The detergent article according to any of the preceding claims, wherein the detergent composition further comprises an additional enzyme selected from the group consisting of proteases, amylase, cellulase, lipases, xyloglucanases, mannanases, nucleases, pectate lyases, and a mixture thereof, preferably wherein the additional enzyme comprises at least a protease.

14. The detergent article according to the preceding claim wherein the article comprises at least two compartments, a first compartment comprising the metalloprotease and a second compartment comprising a further enzyme.

15. The detergent article according to any of the preceding claims, wherein the detergent composition further comprises an alkanolamine selected from the group comprising of monoethanolamine, diethanolamine, triethanolamine and a mixture thereof, preferably monoethanolamine.

16. The detergent article according to any of the preceding claims wherein the detergent composition has a pH of from 6 to 10 as measured in a 10% by weight demineralized water solution at 20°C.

Europäisches Patentamt
European Patent Office
Office européen des brevets

# EUROPEAN SEARCH REPORT

Application Number
EP 24 19 7310

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/279571 A1 (HOUCKHAM LAURA [GB] ET AL) 22 August 2024 (2024-08-22)<br>* paragraphs [0033], [0063], [0066], [0221] - [0223]; table 2 *<br>----- | 1-16 | INV.<br>C11D1/83<br>C11D3/386<br>C11D17/04 |
| X<br><br>Y | US 2016/355767 A1 (SOUTER PHILIP FRANK [GB] ET AL) 8 December 2016 (2016-12-08)<br>* paragraphs [0001] - [0003], [0019] - [0023], [0045], [0028], [0086] - [0106], [0124] - [0126], [0137] - [0129]; claim 2; examples; table 1 *<br>----- | 1-5,7,8,<br>15<br>9-12 | ADD.<br>C11D1/14<br>C11D1/22<br>C11D1/29<br>C11D1/72 |
| Y,D | WO 2007/044993 A2 (GENENCOR INT [US]; PROCTER & GAMBLE [US] ET AL.)<br>19 April 2007 (2007-04-19)<br>* page 1, paragraph 1 - page 3, last paragraph; sequence 18 *<br>* page 7, line 11 - page 9, line 1 *<br>* page 16, paragraph 1 *<br>* page 51, line 174 - page 53, line 7 *<br>* claims 13, 17-18 *<br>* examples 22-24 *<br>----- | 9-12 | TECHNICAL FIELDS SEARCHED (IPC)<br>C11D |
| Y,D | WO 2014/194034 A2 (DANISCO US INC [US])<br>4 December 2014 (2014-12-04)<br>* sequence 33 *<br>----- | 11 | |

The present search report has been drawn up for all claims

| Place of search | Date of completion of the search | Examiner |
|---|---|---|
| The Hague | 31 January 2025 | Marttin, Emmeline |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 19 7310

This annex lists the patent family members relating to the patent documents cited in the above-mentioned European search report. The members are as contained in the European Patent Office EDP file on
The European Patent Office is in no way liable for these particulars which are merely given for the purpose of information.

31-01-2025

| Patent document cited in search report | Publication date | Patent family member(s) | Publication date |
|---|---|---|---|
| US 2024279571 A1 | 22-08-2024 | CN 118119692 A | 31-05-2024 |
| | | EP 4416255 A1 | 21-08-2024 |
| | | US 2024279571 A1 | 22-08-2024 |
| | | WO 2023064749 A1 | 20-04-2023 |
| US 2016355767 A1 | 08-12-2016 | AR 104893 A1 | 23-08-2017 |
| | | CA 2986804 A1 | 08-12-2016 |
| | | CN 107690477 A | 13-02-2018 |
| | | EP 3101103 A1 | 07-12-2016 |
| | | JP 6695906 B2 | 20-05-2020 |
| | | JP 2018516304 A | 21-06-2018 |
| | | RU 2678696 C1 | 31-01-2019 |
| | | US 2016355767 A1 | 08-12-2016 |
| | | WO 2016196705 A1 | 08-12-2016 |
| WO 2007044993 A2 | 19-04-2007 | AR 055444 A1 | 22-08-2007 |
| | | AU 2006299783 A1 | 19-04-2007 |
| | | BR PI0617392 A2 | 26-07-2011 |
| | | CA 2624977 A1 | 19-04-2007 |
| | | CN 101341248 A | 07-01-2009 |
| | | CN 105200027 A | 30-12-2015 |
| | | DK 1934340 T3 | 16-06-2014 |
| | | DK 2390321 T3 | 23-02-2015 |
| | | EP 1934340 A2 | 25-06-2008 |
| | | EP 2390321 A1 | 30-11-2011 |
| | | ES 2529815 T3 | 25-02-2015 |
| | | HK 1127368 A1 | 25-09-2009 |
| | | JP 5507843 B2 | 28-05-2014 |
| | | JP 6677629 B2 | 08-04-2020 |
| | | JP 2009511072 A | 19-03-2009 |
| | | JP 2014064562 A | 17-04-2014 |
| | | JP 2016019518 A | 04-02-2016 |
| | | JP 2017121234 A | 13-07-2017 |
| | | KR 20080066921 A | 17-07-2008 |
| | | KR 20140027423 A | 06-03-2014 |
| | | TW 200730628 A | 16-08-2007 |
| | | US 2008293610 A1 | 27-11-2008 |
| | | US 2009263882 A1 | 22-10-2009 |
| | | US 2012107907 A1 | 03-05-2012 |
| | | US 2014134708 A1 | 15-05-2014 |
| | | US 2017022490 A1 | 26-01-2017 |
| | | US 2018066244 A1 | 08-03-2018 |
| | | US 2020056165 A1 | 20-02-2020 |
| | | US 2022033798 A1 | 03-02-2022 |
| | | US 2024401017 A1 | 05-12-2024 |
| | | WO 2007044993 A2 | 19-04-2007 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 24 19 7310

This annex lists the patent family members relating to the patent documents cited in the above-mentioned European search report. The members are as contained in the European Patent Office EDP file on
The European Patent Office is in no way liable for these particulars which are merely given for the purpose of information.

31-01-2025

| Patent document cited in search report | Publication date | Patent family member(s) | Publication date |
|---|---|---|---|
| WO 2014194034 A2 | 04-12-2014 | CN 105492603 A | 13-04-2016 |
| | | DK 3110833 T3 | 06-04-2020 |
| | | EP 3110833 A2 | 04-01-2017 |
| | | EP 3636662 A1 | 15-04-2020 |
| | | EP 4159854 A1 | 05-04-2023 |
| | | JP 6808675 B2 | 06-01-2021 |
| | | JP 2016526880 A | 08-09-2016 |
| | | JP 2018153183 A | 04-10-2018 |
| | | JP 2020072671 A | 14-05-2020 |
| | | US 2016122738 A1 | 05-05-2016 |
| | | US 2018087038 A1 | 29-03-2018 |
| | | US 2020291374 A1 | 17-09-2020 |
| | | WO 2014194034 A2 | 04-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015158723 A
- WO 2015193488 A
- WO 2014029819 A
- WO 2014029820 A
- WO 2016075078 A
- WO 2014029821 A
- WO 2019105675 A
- WO 2019142774 A
- JP 7057140 B
- US 20080293610 A1
- WO 2007044993 A
- WO 2009058661 A
- US 20140315775 A
- WO 2014194034 A
- WO 2014194117 A
- WO 2004067737 A
- WO 2015091989 A
- WO 2015091990 A
- WO 2015024739 A
- WO 2015143360 A
- US 6312936 B1
- US 5679630 A
- US 4760025 A
- WO 03055974 A
- WO 03054185 A
- WO 03054184 A
- WO 2017215925 A
- DE 102006022216
- WO 2015089447 A
- WO 2015089441 A1
- WO 2016066756 A
- WO 2016066757 A
- WO 2016069557 A
- WO 2016069563 A
- WO 2016069569 A
- WO 2016174234 A
- WO 2017089093 A
- WO 2020156419 A
- WO 2016183509 A
- DE 102006022224 A1
- WO 2020221578 A
- WO 2020221579 A
- WO 2020221580 A
- WO 8906270 A
- WO 05052161 A
- WO 05052146 A
- WO 9217577 A
- WO 0037627 A
- WO 08010925 A
- WO 2011072117 A
- US 5352604 A


### Non-patent literature cited in the description


- **RAWLINGS et al.** *Biochem. J.*, 1993, vol. 290, 205-218
- **RAWLINGS et al.** MEROPS: the peptidase database. *Nucl Acids Res*, 2006, vol. 34, D270-272
- M23 metalloprotease family according to Proteolysis in Cell Function. IOS Press, 1997, 13-21
- M35metalloprotease family according to Proteolysis in Cell Function. IOS Press, 1997, 13-21